# EUROPEAN PATENT APPLICATION

(11) **EP 4 851 293 A1**
(43) Date of publication of application: **09.12.2026**
(21) Application number: 25181372.1
(22) Date of filing: 06.06.2025
(51) Int. Cl.: C12N 15/85, G01N 33/50, G01N 15/14

(54) **CO-CULTURE-BASED HIGH-THROUGHPUT GROWTH RATE ENGINEERING**

(71) Applicant: Sartorius Stedim Cellca GmbH, 89081 Ulm (DE)
(72) Inventor: Marzluf, Jannis, 89081 Ulm (DE); Leroux, Ann-Cathrin, 89081 Ulm (DE); Zehe, Christoph, 89081 Ulm (DE)
(74) Representative: Eisenführ Speiser

(57) **Abstract**

The invention relates to a method for determining the cell growth difference of a target cell population by performing a reciprocal read-out co-culture with at least one control cell population. In particular, the invention relates to a method comprising the co-culture of a target cell population and at least one control cell population, wherein the control cell population expresses at least one marker, preferably a fluorescent marker, enabling quantification of relative cell growth. The invention further relates to methods for screening at least one clone with increased growth rate within the target cell population, a clone obtainable by such methods, and a kit for performing the method. Additionally, the invention relates to the use of the method, the clone, and the kit for the identification and establishment of cell lines with improved growth properties for the production of recombinant molecules.

## Description

### Technical Field

The invention relates to a method for determining the cell growth difference of a target cell population by performing a reciprocal read-out co-culture with at least one control cell population. In particular, the invention relates to a method comprising the co-culture of a target cell population and at least one control cell population, wherein the control cell population expresses at least one marker, preferably a fluorescent marker, enabling quantification of relative cell growth. The invention further relates to methods for screening at least one clone with increased growth rate within the target cell population, a clone obtainable by such methods, and a kit for performing the method. Additionally, the invention relates to the use of the method, the clone, and the kit for the identification and establishment of cell lines with improved growth properties for the production of recombinant molecules.

### Background of the Invention

Cell growth assays form a cornerstone of modern cell biology, underpinning the study of cellular processes, the screening of therapeutic compounds, and the investigation of molecular mechanisms (Schenone et al., 2013). Accurate measurement of cell proliferation rates is critical for these applications, with various methodologies developed over the years to quantify cell growth. Among these are colorimetric assays such as the Tetrazolium Dye (MTT) assay, the Cell Counting Kit-8 (CCK-8) assay, and direct cell counting techniques, both manual and automated (Präbst et al., 2017). Additionally, gross defects in cell proliferation can typically be monitored using well-established viability assays, including trypan blue exclusion or nuclear staining with dyes such as 4',6-diamidino-2-phenylindole (DAPI) or Hoechst 33342. In addition, metabolic assays measuring ATP content via reporter luciferase assays provide an indirect assessment of viable cell numbers over time. However, these approaches are generally optimized for detecting substantial growth impairments and lack the sensitivity to resolve subtle growth phenotypes.

In experimental contexts where comparative growth assessments are required, for example, between drug-treated and control cell groups, these assays demand stringent control of initial seeding densities. Even slight deviations in initial cell numbers can introduce sampling biases, leading to potential misinterpretation of subtle growth effects (Dumont, 1991). This challenge is magnified when detecting minor differences in growth rates, where traditional methods lack the precision and reproducibility necessary to discern biologically relevant variations.

The accurate determination of growth rates is particularly critical in the context of large-scale gene perturbation screens, such as CRISPR-based gene knockout (KO) studies. These screens aim to identify genetic factors influencing cellular proliferation. However, standard methods for viable cell counting often suffer from high noise levels, especially in high-throughput settings constrained by the accuracy and precision of available cell counting devices. Furthermore, while knocking out a single gene may not cause an immediate growth defect but can trigger cascading effects that only gradually impact proliferation. Without sensitive measurement, these minor yet biologically significant changes may go undetected. In engineering CHO cells, small growth disadvantages can accumulate, particularly when balancing productivity with viability or pursuing genome reduction. Subtle fitness costs, if overlooked, may compromise cell line stability, product quality, or performance under bioprocessing conditions.

A further challenge in optimizing cell growth arises from the variability introduced by different recombinant molecules, such as proteins. Each protein imposes distinct demands on the host cell's folding, processing, and secretion machinery, which may lead to protein-specific metabolic burdens. As a result, cellular conditions that support optimal growth for one recombinant product may not be suitable for another. To identify the best conditions and/or metabolic modifications thus requires balancing cell proliferation with the unique biosynthetic stress imposed by each protein, clearly necessitating a flexible and precise screening approach to tailor growth environments for diverse therapeutic targets.

Sensitive assays are therefore essential to identify modifications that maintain growth and robustness while achieving desired traits, ensuring that functionally relevant phenotypes are not missed in large-scale CRISPR screens. Consequently, the detection of modest changes in growth rates remains a technical hurdle in large-scale functional genomics studies.

Eekels et al. present a method in which cells are stably transduced with lentiviral vectors encoding both GFP and a shRNA. The impact of the shRNA on cell proliferation is inferred from changes in the proportion of GFP-positive cells within a mixed culture. However, this design presents several critical disadvantages. Notably, GFP is co-expressed with the shRNA, meaning that any toxic effect, whether due to the shRNA, the integration event, or the marker itself, directly affects GFP⁺ cells. This coupling confounds interpretation: a decline in GFP⁺ cells could result from knockdown effects, general vector toxicity, or stress from overexpression, making it impossible to isolate the effect of the shRNA alone. Further, the use of lentiviral transduction introduces random integration into the genome, which is inherently stochastic. Such variability creates genetic heterogeneity and increases the risk of insertional mutagenesis, distorting proliferation outcomes independently of the shRNA's intended function. Moreover, the untransduced GFP⁻ cells used as controls are not isogenic. They differ not only in shRNA expression but also in their lack of vector exposure and transduction-associated cellular stress. This makes them inappropriate comparators, as observed growth differences may stem from unequal cellular states rather than the test variable. High transduction efficiencies can lead to multiple integrations per cell, intensifying off-target or toxic effects. Even scrambled shRNA controls showed toxicity under these conditions, indicating the system's sensitivity to expression load rather than specific knockdown activity.

In sum, the method by Eekels et al. suffers from entangled variables, non-equivalent controls, and unpredictable integration, which severely limits its interpretability and making it unsuitable for applications requiring the precise and reproducible determination of cellular growth difference.

Song et al. present a modified version of the competitive co-culture assay as used by Eekels et al., introducing the *ComProliM Index* (CPMI) as a linear regression-based metric to assess relative cell proliferation. The only substantive methodological changes are the introduction of a new analytical metric (CPMI) for assessing growth dynamics, and the separation of genetic modification (e.g., shRNA-mediated knockdown) from GFP labelling, whereas Eekels et al. used a single vector co-expressing both shRNA and GFP in the same cell population. However, both methods rely on lentiviral transduction, which introduces random genomic integration of constructs. In Song's system, both test and control cells undergo transduction, e.g., one for shRNA knockdown and the other for SYPL1 overexpression, but the integration sites and copy numbers remain uncontrolled, and the system lacks any use of a CRISPR as a tool for highly specific and controllable genomic modifications. As a result, observed proliferation differences could still stem from vector-related artefacts rather than the specific gene function. Moreover, Song et al. exclusively use adherent cancer cell lines, which are not representative of suspension-adapted production systems and are poorly suited for scalable, automated, or high-throughput applications. While CPMI reduces sensitivity to initial seeding variation, it introduces new dependencies on consistent dilution rates and confluency thresholds, which themselves are difficult to standardize in adherent cultures.

In essence, the conceptual distance between the Eekels and Song methods is minimal. Apart from CPMI and the decoupling of GFP and shRNA transduction, the underlying approach and limitations remain largely the same. Thus, there is an urgent and growing need for sensitive, scalable, and reproducible methods to detect subtle differences in cell growth rates, particularly in the context of genome engineering and functional genomics. Advances in gene editing technologies, such as CRISPR/Cas9, have enabled high-throughput perturbation of cellular genomes, creating unprecedented opportunities for systematically interrogating gene function. However, the ability to translate genetic perturbations into meaningful phenotypic readouts, especially for complex traits like cell proliferation, remains constrained by the limitations of existing assays.

Traditional methods for measuring cell growth, including direct cell counting, metabolic assays, and viability staining, are often hampered by experimental noise, limited sensitivity, and insufficient reproducibility across replicates and platforms. These challenges are magnified in large-scale screening contexts, where small but biologically significant growth effects can be obscured by technical variability. The detection of modest proliferation differences is particularly critical in applications such as identifying non-obvious growth regulators, engineering subtle fitness improvements, or implementing genome reduction strategies that seek to streamline cellular functions without compromising viability.

Moreover, scalable approaches must be compatible with industrially relevant production cell lines, such as Chinese Hamster Ovary (CHO) cells, which are used extensively in the manufacture of recombinant proteins. Methods optimized for adherent or non-production cell types do not readily translate to suspension cultures or high-density bioprocessing conditions, limiting their applicability in biopharmaceutical development. There is also a need to avoid the use of viral vectors and complex reporter systems that can introduce unintended genomic effects, complicate regulatory compliance, and increase production costs.

### Summary of the Invention

The above identified objects are addressed by the technical teaching as provided with the present invention.

A first aspect relates to a method of determining cell growth difference of a target cell population by performing a reciprocal read-out cell co-culture relative to at least one control cell population, comprising the following steps: (i) providing the target cell population and the at least one control cell population as a co-culture; (ii) expressing, preferably constitutively expressing, in the at least one control cell population at least one marker, preferably a fluorescent marker; (iii) co-transfecting the target cell population and the at least one control cell population with at least one molecular stimulant, independently selected from a nucleic acid molecule, including a plasmid DNA, a viral vector, an RNA, an antisense oligonucleotide, a protein, a peptide, a small molecule drug, a growth factor, a cytokine, or a combination thereof, wherein the at least one molecular stimulant induces at least one genomic modification in the target cell population, preferably wherein the at least one molecular stimulant is at least one sgRNA complementary to at least one target sequence in the genome of the at least one target cell population, wherein the at least one target cell population expresses at least one Cas endonuclease and/or a variant thereof; (iv) incubating the cell co-culture, including at least one cell passage, more preferably at least two, more preferably at least three, more preferably at least four, more preferably at least five, including at least 10, at least 20, or at least 30 cell passages; (v) determining cell growth difference of the target cell population based on the measured marker-positive cells at a given time point post-transfection and the initial percentage of marker-positive cells at the time point of co-transfection, preferably by microscopy, live cell imaging and/or flow cytometry, preferably wherein the marker is a fluorescent marker; (vi) optionally: obtaining a clone from the target cell population showing an increased cell growth in comparison to a control cell.

One embodiment relates to the method according to the first aspect, wherein the target cell population constitutively or inducibly encodes at least one recombinant target molecule, preferably at least one recombinant target protein.

One embodiment relates to the method according to the first aspect or the embodiment thereof, wherein the target cell population and the at least one control cell population are co-cultivated at an equivalent initial seeding density.

One embodiment relates to the method according to the first aspect or any embodiment thereof, wherein both the target cell population and the at least one control cell population are eukaryotic cell populations, preferably Chinese Hamster Ovary (CHO) cell populations.

One embodiment relates to the method according to the first aspect or any embodiment thereof, wherein the at least one control cell population expresses a fluorescent marker, preferably a green-fluorescent protein (GFP), or a variant thereof, and the target cell population expresses a Cas endonuclease, or a variant thereof.

One embodiment relates to the method according to the first aspect or any embodiment thereof, wherein the measurement accuracy of cell growth difference of the target cell population positively correlates with the cultivation time and/or number of cell passages relative to the at least one control cell population, according to the following relationship (a) a minimum measurable cell growth difference of at least 30% after a cultivation time of at least three days, preferably at least four days, and/or after at least one cell passage, preferably at least two cell passages; or (b) a minimum measurable cell growth difference of at least 20% after a cultivation time of at least five days, preferably at least six days and/or after at least two cell passages, preferably at least three cell passages; or (c) a minimum measurable cell growth difference of at least 10% after a cultivation time of at least eight days, preferably at least 11 days and/or after at least seven cell passages, preferably at least five cell passages; or (d) a minimum measurable cell growth difference of at least 5% after a cultivation time of at least 15 days, preferably at least 20 days and/or after at least seven cell passages, preferably at least nine cell passages; or (e) a minimum measurable cell growth difference of at least 4% after a cultivation time of at least 19 days, preferably at least 25 days and/or after at least eight cell passages, preferably at least 11 cell passages; or (f) a minimum measurable cell growth difference of at least 3% after a cultivation time of at least 25 days, preferably at least 33 days and/or after at least 11 cell passages, preferably at least 15 cell passages; or (g) a minimum measurable cell growth difference of at least 2% after a cultivation time of at least 38 days, preferably at least 49 days and/or after at least 17 cell passages, preferably at least 22 cell passages; or (h) a minimum measurable cell growth difference of at least 1% after a cultivation time of at least 63 days, preferably at least 97 days and/or after at least 32 cell passages, preferably at least 43 cell passages.

One embodiment relates to the method according to the first aspect or any embodiment thereof, wherein the cell co-culture is maintained in a high-throughput manner, preferably based on an automated well-plate system.

One embodiment relates to the method according to the first aspect or any embodiment thereof, wherein the target cell population and the at least one control cell population are co-transfected by high-throughput electroporation.

A second aspect relates to a method of screening for at least one clone within target cell population, wherein the at least one clone has an increased cell growth difference, wherein the method comprises performing a reciprocal read-out cell co-culture as defined in any of the previous claims to determine at least one clone having an increased cell growth difference; wherein step (iii) of the first aspect additionally comprises an initial step of selecting at least one molecular stimulant suitable to induce increased cell growth of the target cell population, in particular inducing increased cell growth of the target cell population.

A third aspect relates to a clone from the target cell population obtained or obtainable by the method according to the first aspect or any embodiment thereof, or obtained or obtainable by the method according to the second aspect, preferably wherein the target cell population exhibits an cell growth difference rate of at least 50%, 40%, 30%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, compared to the at least one control cell population.

One embodiment relates to the clone according to the third aspect, wherein at least one genomic modification in the target cell population allows for an optimized production of the at least one recombinant target molecule, preferably a recombinant target protein, and wherein said optimized production of the at least one recombinant target protein is characterized by an increase (a) in the yield of the at least one recombinant protein in comparison to a reference, and/or (b) in the yield of the at least one RNA encoding said at least one recombinant protein in comparison to a reference.

A fourth aspect relates to a kit for determining cell growth of a target cell population by performing a reciprocal read-out cell co-culture with at least one control cell population comprising: (i) optionally: a chemically defined cell culture medium for CHO cell growth and transfection by electroporation; (ii) at least one plasmid encoding at least one fluorescent protein, preferably GFP, under a constitutive promoter for stable expression in control cells; (iii) a pre-validated sgRNA library targeting genes known to influence CHO cell growth; (iv) at least one flow cytometry calibration standard; and (v) optionally: detailed instructions for co-culturing in 96-well plates, electroporating using specified parameters, analyzing cell growth via flow cytometry, and calculating relative growth rates using provided software.

A fifth aspect relates to a use of the method according to the first aspect or any embodiment thereof, or according to the method according to the second aspect for determining cell growth of a target cell population by performing a reciprocal read-out cell co-culture with at least one control cell population.

A sixth aspect relates to a use of the kit according to the fourth aspect for determining cell growth of a target cell population by performing a reciprocal read-out cell co-culture with at least one control cell population.

A seventh aspect relates to a use of the clone according to the third aspect or the embodiment thereof for establishing a cell line for the production of a recombinant target molecule, preferably a recombinant target protein, most preferably a therapeutic molecule.

### Brief Description of Figures

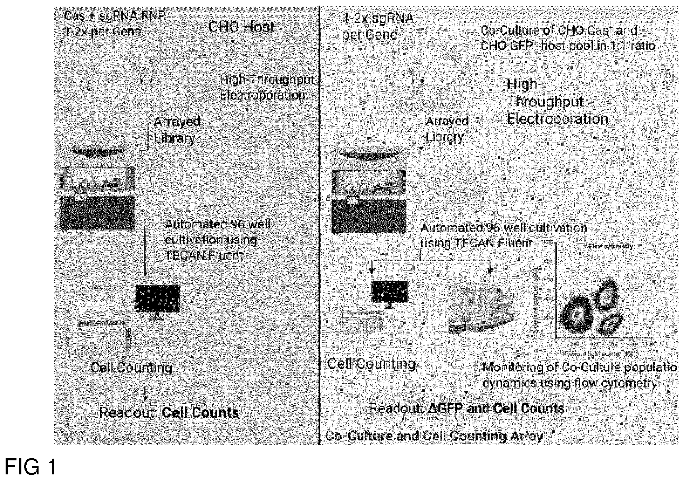

- **FIG 1**: Workflow of traditional vs novel experimental approach for analyzing cell growth in host pools using high-throughput electroporation and automated cultivation. The left panel describes a workflow where Cas endonuclease (Cas) and sgRNA ribonucleoproteins (RNPs) are introduced into CHO cells, followed by automated 96-well cultivation cell counting to measure population changes and passaging. The right panel depicts a co-culture system where CHO Cas9+ and CHO GFP+ cells are electroporated and cultivated together. This setup enables monitoring of population dynamics using flow cytometry, with readouts including GFP fluorescence changes (ΔGFP) and cell counts.

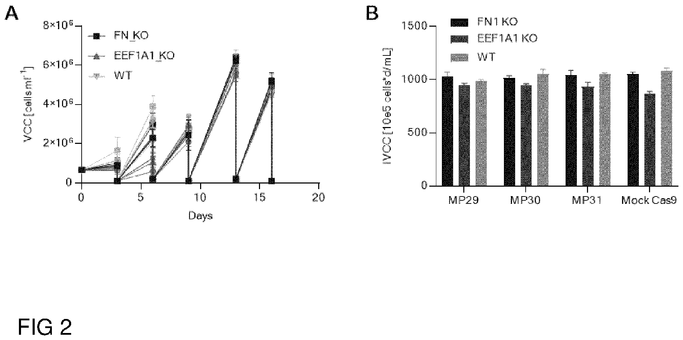

- **FIG 2**: Growth Dynamics of Gene-Edited and Wild-Type Cells. **A:** Cell growth was monitored over time by measuring viable cell concentration (VCC) in cells transfected with FN1-targeting sgRNA (FN1_KO), EEF1A1-targeting sgRNA (EEF1A1_KO), and wild-type (WT) control cells. VCC was assessed at multiple time points over 20 days. **B:** Integral viable cell concentration in different Co-Culture MixPools 29-31 over the time of cultivation. FN1 KO (Blue), EEF1A1 KO (Red) and WT (Green) are shown for each approach.

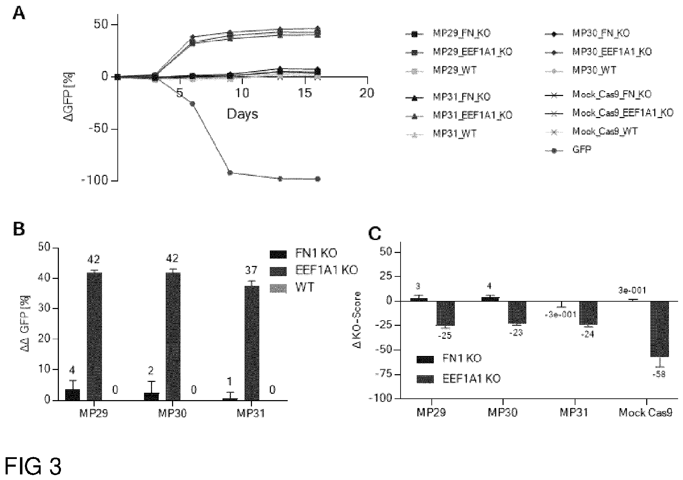

- **FIG 3**: Proof of Concept Co-Culture Screening System. **A:** Change in GFP-positive cell percentage over time in co-culture and control conditions. Experimental groups include unedited wild-type (WT) cells, FN1-targeting sgRNA-transfected cells (FN1_KO), EEF1A1-targeting sgRNA-transfected cells (EEF1A1_KO), and GFP-transfected control cells (GFP). **B:** Quantification of GFP-positive population fraction using the ΔΔGFP method (according to Formula 2 in Example 2). Depicted are changes in FN1- and EEF1A1-targeting sgRNA-transfected pools after two weeks of cultivation. **C:** Change in KO-score over two weeks of cultivation. Depicted are KO-score variations in FN1- and EEF1A1-targeting sgRNA-transfected pools, indicating differential effects on cell population dynamics.

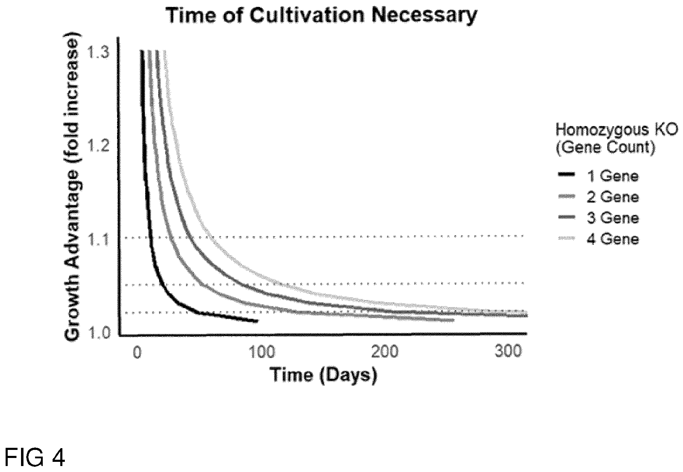

- **FIG 4**: Time of Cultivation Required to Detect Growth Advantages in Co-Culture Pools. Simulated time (in days) required for a faster-growing knockout (KO) variant to reach a detectable abundance (20% change in GFP) in a co-culture system. Each line represents a different gene knockout size (1-4 genes), with starting frequencies derived from experimental mixed pool measurements. A detection threshold was set to 20% of the total population, assuming that such enrichment is sufficient to generate a measurable ΔGFP or barcode shift in pooled screening readouts. Curves demonstrate the nonlinear relationship between growth advantage and time to detection, especially for lower-abundance variants.

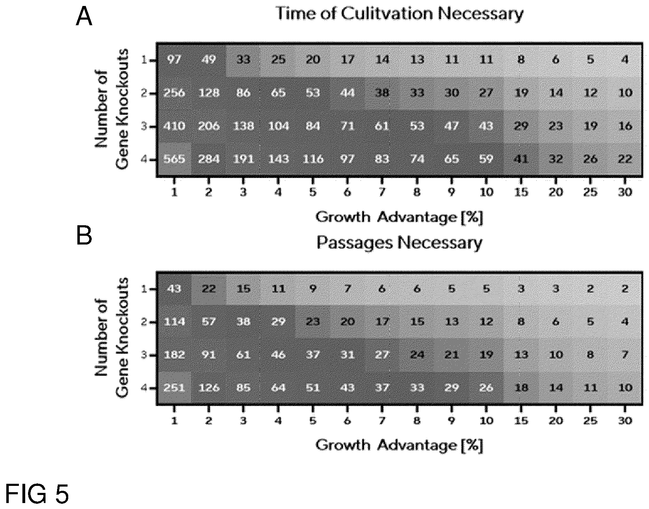

- **FIG 5**: **A:** Heatmap summarizing the number of days required to reach the 20% detection threshold across a range of growth advantages (x-axis, in %) and gene knockout set sizes (y-axis). Warmer colors (darker shading) indicate longer cultivation times. This matrix highlights how small growth advantages and lower initial KO frequencies (as with higher gene KO counts) substantially delay detectability, emphasizing the importance of cultivation time in pooled phenotypic growth screens. **B:** Heatmap showing the estimated number of passages required to reach the same detection threshold. Assuming three population doublings per passage, this representation provides a practical reference for experimental planning, allowing investigators to align expected enrichment dynamics with routine passage schedules. As shown in **A,** detectability improves with increasing growth advantage and higher initial KO abundance.

### Brief Description of Sequences

| **SEQ ID NO:** | **Description** |
|---|---|
| 1 | sgRNA spacer sequence targeting FN1 |
| 2 | sgRNA spacer sequence EEF1A1 |
| 3 | FN1 protein sequence |
| 4 | EEF1A1 protein sequence |

### Definitions

"Average cell growth," within the context of this invention, refers to a quantifiable measure of the overall proliferative capacity and/or metabolic activity of a cell population over a defined period. It is not limited to a single measurement or method but encompasses any approach that provides an indication of how the cell population is increasing in number or biomass. This can include, but is not limited to, direct cell counting (manual or automated), measurements of viable cell concentration, metabolic assays that correlate with cell number (e.g., MTT, CCK-8, ATP assays), assessments of DNA content, or measurements of overall biomass. Furthermore, "average cell growth" can be expressed as a growth rate, doubling time, or any other metric that reflects the rate at which the cell population is expanding or accumulating biomass. The specific method used to determine average cell growth will depend on the cell type, the experimental conditions, and the available equipment. Preferably, average cell growth is determined by the methodology as described in the Examples. "Increased average cell growth," signifies a statistically significant and reproducible improvement in the proliferative capacity and/or metabolic activity of a target cell population compared to a suitable control population, as measured using appropriate assays. This increase is not merely a transient fluctuation but represents a sustained and measurable advantage in cell growth over a defined period. The specific magnitude of the increase considered significant will depend on the experimental context, the sensitivity of the assay used, and the desired outcome, but it must be demonstrably greater than the inherent variability within the system. "Increase average cell growth" can manifest as a higher growth rate, a shorter doubling time, an increased viable cell concentration, or an enhanced metabolic activity, all indicating a more robust and efficient proliferation of the target cell population. The co-culture system described herein provides a sensitive and reliable method for detecting and quantifying such increases in average cell growth.

"Cell growth difference," as used in the context of this invention, refers to a quantifiable disparity in the proliferative capacity and/or metabolic activity of a target cell population when measured relative to at least one control cell population over a defined period. This difference reflects how the target cells grow or accumulate biomass in comparison to the control cells under similar or standardized experimental conditions. It is not limited to a single measurement method but broadly encompasses any scientifically valid approach capable of detecting variation in cell number, viability, or overall metabolic output. Methods for assessing cell growth difference may include, but are not limited to, direct cell counting (manual or automated), measurements of viable cell concentration, metabolic assays that correlate with cell proliferation (such as MTT, CCK-8, or ATP-based assays), assessments of DNA content, or evaluations of total biomass accumulation. The difference may be expressed as an absolute or relative change in growth rate, doubling time, viable cell number, signal intensity from a metabolic assay, or any other metric indicative of cell population expansion.

A "significant cell growth difference" denotes a reproducible and statistically validated deviation that exceeds the inherent variability of the system, representing a genuine biological effect. Such a difference may manifest as either an increase or a decrease in the measured parameter, depending on the experimental design and the nature of the intervention being tested. The magnitude of the difference considered significant will depend on assay sensitivity, the baseline variability of the control population, and the specific objectives of the experimental study.

A "CRISPR/Cas system" refers to a gene editing technology utilized to induce targeted genomic modifications within a cell. This system comprises a Cas Endonuclease (or variant thereof), which is a nuclease enzyme, such as Cas9 or Cas12a, capable of cleaving DNA at a specific target site. The Cas endonuclease may be a Class 2 Type V nuclease, including but not limited to Cas12a (formerly known as Cpf1), MAD7, or Cas14, and may be expressed in only the target cell population. The system also includes a guiding molecule, which is a molecule that directs the Cas endonuclease to the specific target sequence within the genome. This guiding molecule is typically an RNA molecule and may take the form of a single guide RNA (sgRNA), which combines the functions of a CRISPR RNA (crRNA) and a trans-activating CRISPR RNA (tracrRNA) into a single molecule, or a CRISPR RNA (crRNA) that directs the Cas endonuclease (e.g., Cas12a) to the target sequence, or a combination of a crRNA and a tracrRNA. The guiding molecule comprises a nucleotide sequence that is complementary to the target sequence in the genome, thereby enabling the Cas endonuclease to recognize and bind to the target site for cleavage. The CRISPR/Cas system allows for targeted gene knockout, gene editing, or other genomic modifications, thereby influencing cellular characteristics such as growth rate and recombinant protein production. As a molecular stimulant according to the present invention, the CRISPR/Cas system, or components thereof, may be introduced into the target and/or control cell population(s) to induce at least one specific genomic modification in the target cell population. The components of the CRISPR/Cas system may be introduced into the cell via all methods known in the art, including but not limited to plasmid DNA, viral vectors, RNA molecules, or via genomic integration.

The term "expression" refers to the process of transcription (e.g., of a functional guide RNA) and translation (of a protein or polypeptide) within a host cell. The level or degree of expression of a product gene to the cognate RNA in a host cell can be determined on the basis of either the amount of corresponding mRNA that is present in the cell or the amount of the protein encoded by the product gene that is produced by the cell. For example, mRNA transcribed from a product gene is desirably quantitated by northern hybridization (see, e.g., Sambrook et al., Molecular Cloning: A Laboratory Manual, pp. 7.3- 7.57 (Cold Spring Harbor Laboratory Press, 1989). The polypeptide or protein encoded by a product gene can be quantitated either by assaying for the biological activity of the protein or by using assays that are independent of such activity, such as western blotting using antibodies that are capable of reacting with the protein (see, e.g., Sambrook et al., Molecular Cloning: A Laboratory Manual, pp. 18.1-18.88 (Cold Spring Harbor Laboratory Press, 1989).

"Optimized production" refers to the enhancement of recombinant biomolecule expression, synthesis, and processing through genetic, metabolic, and bioprocess engineering strategies. This optimization aims to maximize yield, stability, and functional integrity while improving resource efficiency, scalability, and reproducibility. It is preferably assessed by measuring the relative concentration of the target molecule (e.g., recombinant protein or RNA) in modified CHO cells compared to a reference CHO cell line under standardized conditions. Optimized production can refer to a global or local optimum.

A "recombinant molecule" refers to a nucleic acid or protein that has been artificially modified or engineered through molecular biology techniques to contain genetic sequences not naturally found together. This can involve the insertion, deletion, or alteration of genetic material within a host cell or system.

A "recombinant protein", which is a protein expressed from a genetically engineered nucleic acid sequence, such as a therapeutic monoclonal antibody, an enzyme for industrial applications, or a fluorescent protein (e.g., EGFP). The term "recombinant protein" as used herein, refers to any protein, peptide, or fragment thereof that can be produced upon exogenous introduction into a host cell, typically through recombinant (i.e., foreign) DNA or RNA. This may occur via a plasmid, a viral vector, or as a transgene introduced through genome editing or similar methods. Additionally, a "recombinant protein" may also be encoded by an endogenous gene that has been modified using genome editing technologies, including base editing, prime editing, and insertion/deletion (InDel) mutations induced by genome editing. A "recombinant protein" may serve as a therapeutic protein, peptide, or fragment thereof, or it may be any other functional protein, peptide, or fragment. Examples include antibodies, their variants, or functional fragments, as well as any other prophylactic or therapeutic proteins. Furthermore, any artificially introduced or modified protein, such as a fluorescent protein (e.g., EGFP), is also considered a recombinant protein, provided that the protein or its fragment has been inserted, mutated, or otherwise artificially generated within the host cell of interest.

"Recombinant RNA" refers to an RNA molecule transcribed from recombinant DNA or synthetically engineered RNA sequences, including mRNA vaccines, guide RNAs for CRISPR-based genome editing, or RNA therapeutics.

The terms "RNA interference" (RNAi), "RNA silencing," and "gene silencing" are used interchangeably herein to refer to a gene down-regulation (or knockdown) mechanism that has been demonstrated to exist in all eukaryotes. This mechanism was first identified in plants, where it was termed "post-transcriptional gene silencing" (PTGS). In RNAi, small RNA molecules guide specific effector proteins to a target nucleotide sequence through complementary base pairing, leading to the degradation of the target mRNA.

An "RNAi agent" typically consists of sense and antisense sequences, which are complementary to each other and arranged in reverse orientation within a nucleic acid sequence. When a nucleic acid construct contains both sense and corresponding antisense sequences, their transcription results in an RNA double-stranded structure, often forming an RNA hairpin. In an RNA hairpin, the sense and antisense sequences pair to form a double-stranded region, while an intervening intron loop sequence separates them, forming the loop of the hairpin structure. An RNAi agent may also contain multiple sense-antisense pairs, resulting in the formation of multiple hairpin loops.

The term "target sequence" is defined as a nucleotide sequence, typically a DNA sequence, which can be subjected to modification using a site-directed endonuclease.

A "therapeutic molecule" or refers to a recombinant protein as defined above, or any other recombinant nucleic acid molecule that has a prophylactic or curative effect when used to treat a subject in need thereof.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/emboss_water/) nucleic acids or the EMBOSS Water Pairwise Sequence Alignments (protein) programme (https://www.ebi.ac.uk/jdispatcher/psa/emboss_water) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and SMITH, T.F. 5 & WATERMAN, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

### Detailed Description

As specified in the background section, there is a critical unmet need for a simple, scalable, and precise assay capable of detecting subtle differences in cell growth rates with high reproducibility and minimal experimental bias. The present invention solves these challenges by introducing a reciprocal read-out co-culture system.

To address the above mentioned need, the present invention thus provides in a first aspect relates to a method of determining cell growth difference of a target cell population by performing a reciprocal read-out cell co-culture relative to at least one control cell population, comprising the following steps: (i) providing the target cell population and the at least one control cell population as a co-culture; (ii) expressing, preferably constitutively expressing, in the at least one control cell population at least one marker, preferably a fluorescent marker; (iii) co-transfecting the target cell population and the at least one control cell population with at least one molecular stimulant, independently selected from a nucleic acid molecule, including a plasmid DNA, a viral vector, an RNA, an antisense oligonucleotide, a protein, a peptide, a small molecule drug, a growth factor, a cytokine, or a combination thereof, wherein the at least one molecular stimulant induces at least one genomic modification in the target cell population, preferably wherein the at least one molecular stimulant is at least one sgRNA complementary to at least one target sequence in the genome of the at least one target cell population, wherein the at least one target cell population expresses at least one Cas endonuclease and/or a variant thereof; (iv) incubating the cell co-culture, including at least one cell passage, more preferably at least two, more preferably at least three, more preferably at least four, more preferably at least five, including at least 10, at least 20, or at least 30 cell passages; (v) determining cell growth difference of the target cell population based on the measured marker-positive cells at a given time point post-transfection and the initial percentage of marker-positive cells at the time point of co-transfection, preferably by microscopy, live cell imaging and/or flow cytometry, preferably wherein the marker is a fluorescent marker; (vi) optionally: obtaining a clone from the target cell population showing an increased cell growth in comparison to a control cell.

This method may utilize a mixed pool of two distinct cell populations: one population stably expressing a marker protein, such as green fluorescent protein (GFP), and a second population stably expressing a CRISPR-associated endonuclease, or a variant thereof. By co-transfecting both populations with a molecular stimulant, such as a single-guide RNA (sgRNA), gene knockout is selectively induced in the Cas-expressing, marker-negative cells, while the GFP-expressing cells remain unaffected. Advantageously, this system may leverage changes in relative cell growth of at the control cell population as an indirect reporter for relative growth changes in the marker-negative target cell population. Over successive cell passages, differences in growth rates between the two populations manifest as incremental shifts in the proportion of marker-positive cells within the culture. This cumulative effect allows even small differences in proliferation to become measurable over time, thereby circumventing the inherent noise and variability associated with direct cell counting methods. Notably, the present invention enables high-throughput, arrayed CRISPR/Cas screening for genetic regulators of cell growth. By providing a highly sensitive and internally controlled assay, the invention facilitates the identification of cellular genes that influence growth behaviour, supports growth rate engineering efforts, and enhances the reliability of downstream biological assays where cell proliferation is a critical variable. Moreover, the present invention embodies a combined integration of CRISPR-mediated gene knockout, marker-based co-culture, and longitudinal analysis of cell growth. This approach is particularly well-suited for high-content and scalable screening platforms, where precision, reproducibility, and throughput are essential.

One advantage of the reciprocal read-out co-culture system lies in its unparalleled sensitivity for detecting subtle growth differences. By continuously co-culturing target and control cell populations and meticulously tracking their relative proportions over time, the method allows for the measurement of even minute variations in growth with extreme precision. This is because the effects of even minimal differences in proliferation or fitness accumulate over multiple cell passages, enabling the revelation of subtle growth advantages or disadvantages that would often go undetected by traditional endpoint assays. In addition, by co-transfecting the target cell population and the at least one control cell population with at least one molecular stimulant artifacts created by the transfection or molecular stimulant itself are normalized, avoiding biases created by selective exposure of the test cell population to the molecular stimulant but not the control population or a different molecular stimulant.

Certain embodiments relate to a method of determining average cell growth of a target cell population by performing a reciprocal read-out cell co-culture relative to at least one control cell population, comprising the following steps: (i) providing the target cell population and the at least one control cell population as a co-culture; (ii) expressing, preferably constitutively expressing, in the at least one control cell population at least one marker, preferably a fluorescent marker; (iii) co-transfecting the target cell population and the at least one control cell population with at least one molecular stimulant, independently selected from a nucleic acid molecule, including a plasmid DNA, a viral vector, an RNA, an antisense oligonucleotide, a protein, a peptide, a small molecule drug, a growth factor, a cytokine, or a combination thereof, wherein the at least one molecular stimulant induces at least one genomic modification in the target cell population, preferably wherein the at least one molecular stimulant is at least one sgRNA complementary to at least one target sequence in the genome of the at least one target cell population, wherein the at least one target cell population expresses at least one Cas endonuclease and/or a variant thereof; (iv) incubating the cell co-culture, including at least one cell passage, more preferably at least two, more preferably at least three, more preferably at least four, more preferably at least five, including at least 10, at least 20, or at least 30 cell passages; (v) determining average cell growth of the target cell population based on the measured marker-positive cells at a given time point post-transfection and the initial percentage of marker-positive cells at the time point of co-transfection, preferably by microscopy, live cell imaging and/or flow cytometry, preferably wherein the marker is a fluorescent marker; (vi) optionally: obtaining a clone from the target cell population showing an increased cell growth in comparison to a control cell. The herein disclosed embodiments of the method of determining cell growth difference of a target cell population by performing a reciprocal read-out cell co-culture relative to at least one control cell population are applicable to the embodiments of the method of determining average cell growth of a target cell population by performing a reciprocal read-out cell co-culture relative to at least one control cell population.

In certain embodiments of the method, the at least one molecular stimulant causes a difference in cell growth difference, preferably wherein the difference in cell growth difference is caused by the at least one genomic modification.

In certain embodiments of the method, co-culturing may be performed by a method selected from the group consisting of batch cultivation, fed-batch cultivation, perfusion cultivation, and combinations thereof, but not limited thereof.

A "molecular stimulant" encompasses a diverse array of molecules capable of influencing cellular processes, and particularly the growth rate of a target cell population in a co-culture system. These stimulants are introduced into both the target cell population and at least one control cell population to induce specific changes, and these changes can then be monitored and analyzed. The molecular stimulant may comprise at least one nucleic acid molecule including e.g., plasmid DNA, viral vectors, RNA, and antisense oligonucleotides, capable of modifying gene expression. Furthermore, the molecular stimulant may comprise at least one protein and/or peptide, which may act as signaling molecules, and enzymes, and/or structural components capable of modifying cellular behaviour. The molecular stimulant may comprise small molecule drugs. These are typically organic compounds and may interact with specific cellular targets, such as proteins or nucleic acids, to modulate their function. The molecular stimulant may comprise growth factors and cytokines acting as signaling molecules capable of stimulating cell growth and/or metabolic activity.

A key feature of the molecular stimulant is its ability to induce a change within the target cell population, and this change can be a genomic modification, and/or a change in gene expression, or any other alteration and this alteration ultimately affects the cell's growth rate. The effect of the molecular stimulant may be preferably assessed by monitoring the relative growth of the target cell population in comparison to the control cell population within the co-culture system.

In a preferred embodiment of the method, the Cas endonuclease, or variant thereof may be a Class 2 Type V nuclease, or a variant or mutant thereof, including a Cas12a (formerly 5 known as Cpf1) or a MAD7 or a Cas14 effector. Suitable vectors and plasmids to express the relevant CRISPR effector and the cognate gRNA(s) are available to the skilled person and can be easily adapted by designing the gRNA(s) *in silico.*

As it is known to one skilled in the art, CRISPR/Cas effectors need a guiding molecule to exert their activity. The "guide molecule", in particular the "guide RNA" (gRNA) may comprise a trans-activating CRISPR RNA (tracrRNA) and a CRISPR RNA (crRNA). Certain CRISPR nucleases, e.g., Cas12a, only require a crRNA. TracrRNA and crRNA can be combined in one molecule, resulting in a single guide RNA (sgRNA), which comprises the sequence information targeting the genomic target sequence for cleavage by the nuclease.

For multiplexing purposes, more than one gRNA allowing the recognition and thus modification of more than one target site in a genome may be designed and used in one and the same experiment, or one after another.

In certain embodiments, a knock-out of at least one target gene via, for example, an RNAi agent and a knock-out mediated by genome editing, including CRISPR/Cas, may be combined to reduce the expression of the at least one target gene.

Certain embodiments relate to a method for obtaining at least one clone, wherein the at least one clone has an improved growth phenotype due to knock-out of at least one target gene, preferably comprising the following steps: (i) the at least one identified target gene is independently screened by designing a precise CRISPR single-guide RNA set (sgRNA-set) directed to the gene of interest; (ii) the CHO cells are transfected with the sgRNA, followed by expansion under suitable selection; and (iii) the improved growth phenotype is verified in a shake-flask culture or a perfusion bioreactor.

In one embodiment of the method according to the first aspect, the at least one molecular stimulant induces a reduction of expression by RNA interference of at least one target gene in the target cell population, preferably wherein the at least one molecular stimulant is at least one RNAi agent, including but not limited to siRNA, shRNA, or microRNA, complementary to a target sequence of the at least one target gene in the target cell population, wherein the reduction of expression of the at least one target gene results in a change of the cell growth difference.

For RNAi, small RNAs function to guide specific effector proteins to a target nucleotide sequence by complementary base pairing resulting in degradation of the target. A "gene silencing construct" or "RNAi agent" usually comprises so called "sense" and "antisense" sequences. Sense and antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". With these RNA hairpin sequences, more than one target gene can be knocked-down simultaneously in certain embodiments.

A knock-out of at least one target gene via, for example, an RNAi agent and a knock-out mediated by genome editing, including CRISPR/Cas, may be combined to reduce the expression of the at least one target gene.

Advantageously, the method offers a robust and versatile platform for assessing a multitude of positive effects on cellular viability and proliferation. The inherent sensitivity of the reciprocal read-out co-culture system to changes in population dynamics makes it ideally suited for monitoring parameters beyond cellular growth rate. This system can readily be adapted to evaluate metabolic activity or measure resistance to specific stress factors. By incorporating relevant markers or assays, the relative advantage or disadvantage conferred by a molecular stimulant on the viability and/or metabolic activity on target cell population, compared to the control population, can be definitively evaluated for these diverse characteristics.

The "marker," as utilized within the context of this invention, refers broadly to any detectable molecule or characteristic that allows for the distinction and quantification of the control cell population within the co-culture system. While fluorescent proteins, such as GFP, RFP, mCherry, and mScarlet, represent a preferred class of markers due to their ease of detection via flow cytometry and microscopy, the invention is not limited to these. In principle, any marker that is not endogenously present in the cell can be used, provided it can be reliably detected. This includes cell surface markers like CD28, CD, and LNGFR, which can be identified using antibodies or other binding agents. The marker also comprises HA, Myc, FLAG, and Strep-tag, but not limited thereof. Furthermore, the system is not limited to a single control cell population or a single target cell population, as additional cultures can be added to examine more complex interactions. Multiple control cell populations, each with a distinct marker, could be used to assess different aspects of cell growth or to control for potential confounding factors. Similarly, multiple target cell populations, each with a different genetic modification (e.g., different gene knockouts), each labeled with a distinct marker, could be co-cultured to examine combinatorial effects on cell growth or to assess the impact of disrupting specific pathways.

A key requirement for the marker is its ability to provide a clear and quantifiable signal that allows for the accurate determination of the at least one control cell population over time and to enable the assessment of individual or combinatorial effects of molecular stimulants on cell growth and other cellular characteristics.

The determination of cell growth difference within the reciprocal read-out co-culture system can be achieved through a variety of detection methods, with the choice depending on the specific experimental setup and available equipment. While flow cytometry (FACS) using instruments like iQue, MacsQuant, and CytoFLEX is a preferred method for quantifying the relative proportions of marker-positive and marker-negative cells in suspension cultures, the invention is not limited to this technique. Alternative detection methods, such as fluorescence microscopy, are also well-suited for this purpose. In particular, live-cell imaging techniques, utilizing instruments like Incucyte, CellaVista, and Operetta, offer the advantage of real-time monitoring of cell growth and marker expression over extended periods. High-content imaging systems, such as Cytation and ImageXpress, provide automated image acquisition and analysis capabilities, further enhancing the throughput and reproducibility of the method. Standard fluorescence microscopy can also be used for manual or semi-automated quantification of cell populations. For adherent cultures, where passaging may disrupt the cell layer, imaging-based methods are often preferred as they allow for non-invasive monitoring of cell growth and marker expression directly within the culture vessel. For suspension culture, different detection methods can be used, wherein flow cytometry based methods are preferred, as no cell settling step is required and/or the cells in suspension can be directly measured. Hence, according to a preferred embodiment the method comprises determining cell growth difference of the target cell population based on the measured marker-positive cells at a given time point post-transfection and the initial percentage of marker-positive cells at the time point of co-transfection by flow cytometry, preferably wherein the marker is a fluorescent marker.

Certain embodiments relate to the method, wherein step (v) of the first aspect additionally comprises assigning a ΔKO-Score to the target cell population, wherein assigning a ΔKO-Score to the target cell population comprises: (a) extracting the DNA of the target cell population (b) amplifying a genomic region of a gene of the target cell population, preferably by PCR; (c) sequencing the amplified product, preferably by Sanger sequencing;(d) analyzing the sequencing data to quantify the proportion of cells containing at least one genomic modification; and (e) calculating the ΔKO-Score as the difference between the genomically modified fraction at a specified time point (KO-Scoret) and the initial genomically modified fraction measured at a time point post-transfection, preferably at day 2 post-transfection (KO-Score₀). Representative determined ΔKO-Scores are shown in FIG 3C.

Advantageously, the ΔKO Score, determined through PCR amplification, Sanger sequencing, and analysis of indel frequencies at the target site, might offer a direct measure of the proportion of cells harboring functional knockouts at a specific time point. By tracking the ΔKO Score over time in the co-culture system, the method provides insights into the dynamics of gene knockout and its influence on cell growth and fitness. This approach may be particularly advantageous for high-content or scalable platforms, where simpler and more direct readouts are desired.

One embodiment relates to the method, wherein the target cell population constitutively or inducibly encodes at least one recombinant target molecule, preferably at least one recombinant target protein.

In a preferred embodiment of the method according to the present invention, the at least one recombinant target protein may be selected from the group consisting of, but not restricted to, therapeutic proteins, monoclonal antibodies, bispecific antibodies, fusion proteins, peptibodies, and peptides.

One embodiment relates to the method, wherein the at least one recombinant target molecule is a secreted protein or peptide, allowing for the assessment of its production level and/or activity in the co-culture medium, thereby providing a measure of both cell growth and recombinant protein expression.

One embodiment relates to the method, wherein the inducible expression of the at least one recombinant target molecule is controlled by an external stimulus, such as a chemical inducer or a change in temperature, allowing for the temporal control of recombinant protein expression and the assessment of its impact on cell growth under different conditions.

One embodiment relates to the method, wherein the target cell population encodes multiple recombinant target molecules, either constitutively or inducibly, allowing for the assessment of their combined effects on cell growth and/or other cellular characteristics, or enabling the optimization of multi-component recombinant production systems.

One embodiment relates to the method, wherein the target cell population and the at least one control cell population are co-cultivated at an equivalent initial seeding density.

One embodiment relates to the method, wherein the target cell population and the at least one control cell population are co-cultivated at a defined initial seeding ratio, which may be optimized to enhance the sensitivity of the assay for detecting subtle differences in cell growth rates.

One embodiment relates to the method, wherein the target cell population and the at least one control cell population are co-cultivated at non-equivalent initial seeding densities.

One embodiment relates to the method, wherein the initial seeding density or ratio of the target cell population and the at least one control cell population is determined and optimized using an automated system based on preliminary data to maximize assay performance and reproducibility.

One embodiment relates to the method, wherein both the target cell population and the at least one control cell population are eukaryotic cell populations, preferably Chinese Hamster Ovary (CHO) cell populations.

In certain embodiments, the CHO cell population may be selected from, or may be derived from, meaning originating from, a CHO cell variant selected from the group consisting of CHO-K1, CHO-DXB11 (synonymously referred to as CHO-DUKX), CHO-DG44, CHO-S, and CHO-Pro minus, or a CHO GS knockout. Alternatively, the modified CHO cell according to the present invention may also be derived from other CHO cell variants.

In certain embodiments, the modified CHO cell may be, or may be derived from a CHODG44.

In certain embodiments, the modified CHO cell may be, or may be derived from a CHO-K1.

In certain embodiments, the modified CHO cell may be, or may be derived from CHO-DXB11 (synonymously referred to as CHO-DUKX).

In certain embodiments, the CHO cell may be, or may be derived from a CHO GS knock-out cell.

In certain embodiments, the modified CHO cell may be derived from CHO-S or CHO-Pro minus.

One embodiment relates to the method, wherein the at least one control cell population expresses a fluorescent marker, preferably a green-fluorescent protein (GFP), or a variant thereof, and the target cell population expresses a Cas endonuclease, or a variant thereof.

One embodiment relates to the method, wherein the measurement accuracy of cell growth difference of the target cell population positively correlates with the cultivation time and/or number of cell passages relative to the at least one control cell population, according to the following relationship (a) a minimum measurable cell growth difference of at least 30% after a cultivation time of at least three days, preferably at least four days, and/or after at least one cell passage, preferably at least two cell passages; or (b) a minimum measurable cell growth difference of at least 20% after a cultivation time of at least five days, preferably at least six days and/or after at least two cell passages, preferably at least three cell passages; or (c) a minimum measurable cell growth difference of at least 10% after a cultivation time of at least eight days, preferably at least 11 days and/or after at least seven cell passages, preferably at least five cell passages; or (d) a minimum measurable cell growth difference of at least 5% after a cultivation time of at least 15 days, preferably at least 20 days and/or after at least seven cell passages, preferably at least nine cell passages; or (e) a minimum measurable cell growth difference of at least 4% after a cultivation time of at least 19 days, preferably at least 25 days and/or after at least eight cell passages, preferably at least 11 cell passages; or (f) a minimum measurable cell growth difference of at least 3% after a cultivation time of at least 25 days, preferably at least 33 days and/or after at least 11 cell passages, preferably at least 15 cell passages; or (g) a minimum measurable cell growth difference of at least 2% after a cultivation time of at least 38 days, preferably at least 49 days and/or after at least 17 cell passages, preferably at least 22 cell passages; or (h) a minimum measurable cell growth difference of at least 1% after a cultivation time of at least 63 days, preferably at least 97 days and/or after at least 32 cell passages, preferably at least 43 cell passages.

Certain embodiments relate to the method, wherein the measurement accuracy of cell growth difference of the target cell population positively correlates with the cultivation time and/or number of cell passages relative to the at least one control cell population, according to the relationship as indicated in FIG 4 and FIG 5. The skilled person is well aware that in cell culture experiments, error rates in growth measurements, e.g., arising from pipetting inconsistencies, plating variability, and inter-assay noise, typically range between 10% and 25%, depending on the assay format, cell type, and detection method. By extending cultivation time and enabling cumulative signal integration through successive passages, the present method effectively suppresses short-term stochastic fluctuations and converges toward more accurate quantification of relative growth differences.

One embodiment relates to the method, wherein the cell co-culture is maintained in a high-throughput manner, preferably based on an automated well-plate system.

One embodiment relates to the method, further comprising an automated data analysis pipeline that processes the raw data from the high-throughput assay to calculate cell growth rates, identify statistically significant differences, and generate reports, thereby streamlining the data analysis process.

One embodiment relates to the method, wherein the target cell population and the at least one control cell population are co-transfected by high-throughput electroporation.

One embodiment relates to the method, wherein the target cell population and the at least one control cell population are co-transfected using alternative high-throughput transfection methods, such as chemical transfection or viral transduction.

One embodiment relates to the method, wherein the transfection conditions, such as DNA/RNA concentration, transfection reagent, and incubation time, are optimized for each cell type to maximize transfection efficiency and minimize cytotoxicity. Certain embodiments relate to a method, wherein the target cell population and the at least one control cell population are CHO cells, wherein the molecular stimulant comprises a CRISPR/Cas system for inducing genomic modification, wherein cell growth difference is determined via flow cytometry or microscopy by measuring the proportion of cells expressing a fluorescent marker, and wherein the measurement accuracy of cell growth difference of the target cell population positively correlates with the cultivation time and/or number of cell passages relative to the at least one control cell population.

Certain embodiments relate to a method, wherein the co-culture is maintained in suspension culture, wherein the molecular stimulant comprises a CRISPR/Cas system, wherein the cell growth difference is assessed after at least five cell passages, and wherein the measurement accuracy of cell growth difference of the target cell population positively correlates with the cultivation time and/or number of cell passages relative to the at least one control cell population.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth rate difference of at least 30% is achieved after a cultivation time of at least three days, preferably at least four days, and/or after at least one cell passage, preferably at least two cell passages.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth difference of at least 20% is achieved after a cultivation time of at least five days, preferably at least six days, and/or after at least two cell passages, preferably at least three cell passages.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth difference of at least 10% is achieved after a cultivation time of at least eight days, preferably at least 11 days, and/or after at least seven cell passages, preferably at least five cell passages.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth difference of at least 5% is achieved after a cultivation time of at least 15 days, preferably at least 20 days, and/or after at least seven cell passages, preferably at least nine cell passages.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth difference of at least 4% is achieved after a cultivation time of at least 19 days, preferably at least 25 days, and/or after at least eight cell passages, preferably at least 11 cell passages.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth difference of at least 3% is achieved after a cultivation time of at least 25 days, preferably at least 33 days, and/or after at least 11 cell passages, preferably at least 15 cell passages.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth difference of at least 2% is achieved after a cultivation time of at least 38 days, preferably at least 49 days, and/or after at least 17 cell passages, preferably at least 22 cell passages.

Certain embodiments relate to a method, wherein the method utilizes CHO cells, wherein the measurement accuracy of cell growth difference of the target cell population positively correlates with the cultivation time and/or number of cell passages relative to the at least one control cell population, and wherein a minimum measurable cell growth difference of at least 1% is achieved after a cultivation time of at least 63 days, preferably at least 97 days, and/or after at least 32 cell passages, preferably at least 43 cell passages.

A second aspect relates to a method of screening for at least one clone within target cell population, wherein the at least one clone has an increased cell growth difference, wherein the method comprises performing a reciprocal read-out cell co-culture as defined in any of the previous claims to determine at least one clone having an increased cell growth difference; wherein step (iii) of the first aspect additionally comprises an initial step of selecting at least one molecular stimulant suitable to induce increased cell growth of the target cell population, in particular inducing increased cell growth of the target cell population.

The co-culture system can be modified to assess and screen for alterations in other desirable cellular characteristics, such as increased protein production, altered metabolic activity, or enhanced resistance to specific stress factors. By incorporating appropriate markers and assays, the reciprocal read-out approach can be used to identify clones exhibiting the desired alteration. Moreover, the method can be extended to perform multiparameter screening, enabling the simultaneous assessment of multiple cellular characteristics, such as growth rate, protein production, and viability. This allows for the identification of clones exhibiting a desired combination of characteristics, reflecting the complex optimization needs often encountered in biopharmaceutical production and other applications. By identifying molecular stimulants capable of inducing specific combinations of cellular characteristics, the co-culture system can be used to engineer cell lines with tailored properties for a variety of purposes.

A third aspect relates to a clone from the target cell population obtained or obtainable by the method according to the first aspect or any embodiment thereof, or obtained or obtainable by the method according to the second aspect, preferably wherein the target cell population exhibits an cell growth difference rate of at least 50%, 40%, 30%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, compared to the at least one control cell population.

One embodiment relates to the clone, wherein at least one genomic modification in the target cell population allows for an optimized production of the at least one recombinant target molecule, preferably a recombinant target protein, and wherein said optimized production of the at least one recombinant target protein is characterized by an increase (a) in the yield of the at least one recombinant protein in comparison to a reference, and/or (b) in the yield of the at least one RNA encoding said at least one recombinant protein in comparison to a reference.

A key advantage of introducing genomic modifications into the target cell population is the potential to optimize the production of recombinant molecules, particularly recombinant target proteins, and to produce stable cell lines. The genomic modification, achieved through techniques such as CRISPR-Cas9 or other genome editing methods, can be designed to enhance various aspects of recombinant protein production, leading to quantifiable improvements and stable integration of the desired traits. The optimized production of the recombinant target protein can be characterized by an increase in the yield of the recombinant protein, referring to an increase in the total amount of recombinant protein produced per cell or per unit volume of culture, and this can be achieved through various mechanisms, such as increasing the transcription rate of the recombinant gene, improving the stability of the mRNA transcript, enhancing the efficiency of translation or reducing protein degradation. Furthermore, optimized production can be characterized by an increase in the yield of the RNA encoding the recombinant protein, and this genomic modification can also be designed to increase the amount of RNA (mRNA) encoding the recombinant protein, and this can be achieved by optimizing the promoter sequence driving transcription of the recombinant gene, increasing the copy number of the recombinant gene, or improving the processing and stability of the mRNA transcript. It is important to note that the optimized production of the recombinant target protein may involve a combination of both increased RNA yield and increased translational efficiency, leading to a synergistic effect on overall protein production. The specific genomic modification(s) required to achieve optimized production will depend on the specific recombinant protein, the host cell, and the desired level of improvement.

For evaluating optimized production of a recombinant target protein or RNA following at least one genomic modification in a target cell population, a "reference" refers to another target cell population that has not undergone said genomic modification, against which the improved yield is evaluated. Comparing the target cell population to this other cell population allows for determining whether the at least one genomic modification has resulted in an increase in the yield of the at least one recombinant protein or the at least one RNA encoding said at least one recombinant protein, wherein said increase may be determined by any method known in the art.

A fourth aspect relates to a kit for determining cell growth of a target cell population by performing a reciprocal read-out cell co-culture with at least one control cell population comprising: (i) optionally: a chemically defined cell culture medium for CHO cell growth and transfection by electroporation; (ii) at least one plasmid encoding at least one fluorescent protein, preferably GFP, under a constitutive promoter for stable expression in control cells; (iii) a pre-validated sgRNA library targeting genes known to influence CHO cell growth; (iv) at least one flow cytometry calibration standard; and (v) optionally: detailed instructions for co-culturing in 96-well plates, electroporating using specified parameters, analyzing cell growth via flow cytometry, and calculating relative growth rates using provided software.

A fifth aspect relates to a use of the method according to the first aspect or any embodiment thereof, or according to the method according to the second aspect for determining cell growth of a target cell population by performing a reciprocal read-out cell co-culture with at least one control cell population.

A sixth aspect relates to a use of the kit according to the fourth aspect for determining cell growth of a target cell population by performing a reciprocal read-out cell co-culture with at least one control cell population.

A seventh aspect relates to a use of the clone according to the third aspect or the embodiment thereof for establishing a cell line for the production of a recombinant target molecule, preferably a recombinant target protein, most preferably a therapeutic molecule.

The present invention will now be further illustrated with the help of the following nonlimiting Examples.

### Examples

### Example 1: Determining the cell growth difference of a target cell population in co-culture with a control cell population

To investigate the impact of gene knockouts on GFP-positive cell dynamics in mixed co-culture systems, we established three independent mixed pools (MP29, MP30, and MP31) by combining a stable Cas9-expressing CHO host cell line with a GFP-expressing CHO cell pool derived from the same parental line. The GFP-expressing pool was generated by transfecting the parental CHO line with a plasmid encoding GFP, followed by puromycin selection. Multiple puromycin-selected GFP pools were combined to ensure population diversity. To enable precise discrimination between GFP-positive and GFP-negative cells during flow cytometry, fluorescence-activated cell sorting (FACS) was performed based on GFP fluorescence intensity. Specifically, cells were sorted into four subpopulations, low, medium, high, and very high GFP expression, according to predefined gating thresholds. From each subpopulation, 200,000 cells were collected to create a composite GFP+ reference pool. Flow cytometric analyses conducted at regular intervals confirmed long-term stability of the GFP-positive population, with no significant drift in GFP expression over time.

CRISPR-Cas9-mediated knockout studies were designed to evaluate the selective impact of gene disruption within these mixed co-culture pools. Target genes included *FN1,* selected as a non-essential gene with no expected effect on cell proliferation, and *EEF1A1*, selected based on prior evidence suggesting its knockout results in lethality. The hypothesis was that targeting *EEF1A1* would lead to a selective outgrowth of GFP-positive cells, due to the loss of non-GFP cells undergoing growth arrest, while *FN1* knockout would yield no significant change in GFP distribution. To this end, MixPools MP29-MP31 were electroporated with sgRNAs targeting either *FN1 (SEQ ID NO:* 1) or *EEF1A1* (SEQ ID NO: 2) using the Neon Transfection System, and cells were subsequently cultured in 96-well plates under defined seeding conditions using the TECAN Fluent automated liquid handling platform. GFP distribution was monitored at each passage via flow cytometry, and cell viability was assessed by measuring viable cell concentration (VCC) through trypan blue exclusion assays using an automated cell counter. **FIG 2A** depicts the VCC over five serial passages spanning 20 days. In the early phase (days 0-7), wild-type (WT) cells achieved the highest cell densities, followed by *FN1_KO*, with *EEF1A1_KO* pools displaying the lowest VCC, consistent with a transient growth disadvantage upon *EEF1A1* disruption. After day 7, however, cell growth kinetics converged, and final cell densities at each passage showed no significant difference across *WT, FN1_KO,* and *EEF1A1_KO* groups. **FIG 2B** presents integral viable cell concentrations (IVCC) for MP29-MP31, further corroborating these observations. Notably, a pronounced reduction in IVCC was observed in the *EEF1A1* knockout condition within the parental Cas9-expressing line (Mock Cas9), but not in the MixPools. This suggests that in the mixed context, the lethal knockout leads to early depletion of non-GFP cells, after which surviving GFP-positive cells dominate the culture, effectively masking the initial growth suppression.

These data demonstrate that functionally knocked-out, non-GFP cells are selectively removed from the population early during cultivation. As cultures expand, the influence of these non-viable or growth-arrested cells is diluted, resulting in normalization of growth metrics across all conditions in the later phases of the experiment.

### Example 2: Quantification of Population Shifts via GFP Co-Culture Metrics

While overall growth dynamics in mixed co-culture pools did not exhibit significant divergence beyond the second passage, and reductions in integrated viable cell concentration (IVCC) were primarily observed in the Mock Cas9 control condition, these aggregate metrics were insufficient to resolve subtle population shifts within the cultures. Specifically, the progressive loss of EEF1A1-knockout cells was obscured by the proliferation of unaffected cells, thereby limiting the utility of total cell counts in detecting the phenotypic consequences of lethal gene knockout.

To overcome this limitation, population composition was assessed using a GFP-based co-culture system. Quantification of the GFP-positive cell fraction was performed using the ΔGFP [%] metric, defined as the difference between the percentage of GFP-positive cells at a given time point and the corresponding baseline percentage measured on the day of transfection for the same culture. This calculation enabled longitudinal tracking of shifts in GFP expression, serving as a proxy for relative changes in the abundance of the non-GFP knockout population.

To control for potential spontaneous fluctuations in GFP expression in untreated cultures, a secondary normalization metric, ΔΔGFP [%], was introduced. ΔΔGFP was computed by subtracting the ΔGFP value of the untreated wild-type (WT) control culture from the ΔGFP value observed in the treated condition at each respective time point. This normalization accounted for baseline variation and improved the specificity of the assay for detecting true perturbation effects. In alternative cases when high initial CRISPR editing toxicity might be observed in for example multiplexed KO studies, an alternative measure might be implemented calculating the ΔMaxGFP [%] as the fraction of lost GFP calculated from the initial peak after editing by subtracting maximum observed GFP percentage from the final GFP percentage. The mathematical formulations for both ΔGFP, ΔΔGFP and ΔMaxGFP calculations are shown in Formula (1), (2) and (3). $ΔGFP \left[%\right] = {GFP%}_{t} - {GFP%}_{0}$ (1) $ΔΔGFP \left[%\right] = {GFP%}_{treated} - {GFP%}_{WT}$ (2) $ΔMaxGFP \left[%\right] = {GFP%}_{final} - {GFP%}_{max}$ (3)

The *GFP%ₜ* represents the GFP-positive fraction at a given time point, and *GFP%₀* is the initial fraction recorded at the time of transfection of the same culture. This approach ensures that any observed enrichment of GFP-positive cells reflects a treatment-specific effect rather than inherent fluctuations in GFP-positive population. As shown in FIG 3A, the centered time-course analysis of GFP percentage in the mixed pools reveals a pronounced increase in GFP-positive cells over time in the EEF1A1_KO condition. This increase suggests a substantial turnover in population composition, with the loss of non-GFP-expressing cells leading to an enrichment of GFP-positive cells. Further quantification using the normalized ΔΔGFP metric (FIG 3B) demonstrated an absolute increase of 37% to 42% in GFP-positive cells from a starting fraction of 44%, confirming that a significant proportion of the population was replaced over time. This strong enrichment of GFP-expressing cells indicates that non-GFP cells, particularly those transfected with EEF1A1-targeting sgRNAs, were selectively eliminated from the culture due to growth arrest following lethal gene knockout. The ΔKO score (FIG 3C) provides an additional measure of population dynamics by assessing changes in the fraction of EEF1A1-KO cells over time. The KO score is determined through PCR amplification, Sanger sequencing, and Inference of CRISPR Edits (ICE) analysis, which collectively quantify the proportion of cells harboring indels leading to functional KO at the EEF1A1 target site. The ΔKO score is calculated as the difference between the KO score at a given time point and the initial KO score measured on day 2 post-transfection. The *KO_Scoreₜ* represents the knockout fraction at a specific time point, and *KO_Score₀* denotes the initial fraction at the start of the experiment, determined by Formula (4). $ΔKO score = KO _ {Score}_{t} - KO _ {Score}_{0}$ (4)

The ΔKO score closely mirrors the trend observed in the ΔGFP metric, reflecting a consistent decline in EEF1A1-knockout cells over time. Although the absolute magnitude of the ΔKO score is generally lower, this difference is attributable to the complex, multi-step nature of genomic analysis workflows - including DNA extraction, PCR amplification, sequencing, and indel quantification - which collectively introduce some degree of technical variability. In contrast, flow cytometry-based GFP analysis offers a more streamlined and direct measurement of population dynamics, contributing to its relatively lower variance.

Importantly, these findings highlight the complementary strengths of both readouts. While traditional bulk growth metrics such as viable cell concentration (VCC) and integrated viable cell concentration (IVCC) may fail to detect subtle yet biologically meaningful shifts in cell composition, the GFP-based co-culture approach reliably reveals the selective depletion of knockout populations alongside the enrichment of unaffected cells. Together, these results validate the co-culture screening system as a sensitive and robust platform for identifying gene knockouts that alter cellular growth behaviour.

These data demonstrate the system's ability to provide insights into population dynamics, making it possible to observe the effects of KOs on cellular growth independently of viable cell count (VCC) data. The co-culture approach facilitates faster growth rate engineering and multiplex screening, ultimately contributing to more efficient biopharmaceutical production and development workflows.

### Example 3: Simulation of KO Enrichment Dynamics and Detection Sensitivity

To estimate the cultivation duration required for a faster-growing knockout (KO) variant to generate a detectable shift in a co-culture growth-based CRISPR screen (e.g., corresponding to a ΔGFP signal of 20%), a two-pronged simulation strategy was implemented. The objective was to quantify the time necessary to detect phenotypic effects across a range of growth advantages and initial KO cell fractions, using experimentally derived frequencies for homozygous knockouts ranging from single to quadruple gene disruptions.

In the first phase of the analysis, co-culture dynamics were simulated by assigning initial KO cell fractions based on observed distributions within the mixed transfection pool. Exponential growth was assumed, with a fixed multiplicative growth advantage assigned to KO cells relative to wild-type (WT) counterparts. Detectability was operationally defined as the point at which KO cells constituted 20% of the total population - an experimentally conservative threshold corresponding to measurable shifts in GFP or barcode signals. Notably, shifts are often detectable at lower thresholds (5-10%); however, a 20% threshold was chosen to maintain a stringent detection criterion.

For each KO condition (one to four genes disrupted), the number of days required to reach the 20% detection threshold was computed across a range of relative growth advantages (1-30%). As shown in FIG 4, conditions with lower initial KO frequencies (associated with higher-order knockouts) and smaller fitness advantages were found to require significantly extended cultivation periods to surpass the defined detection limit.

The simulation outputs were further visualized as a heatmap (FIG 5A), summarizing the relationship between cultivation time, knockout complexity, and relative growth advantage. A steep, nonlinear dependency was observed, wherein both initial KO frequency and growth rate advantage jointly influenced the time required for detectable enrichment. These findings provide a quantitative framework for optimizing screen duration in relation to expected phenotypic strength and editing efficiency.

To facilitate translation of these findings into routine laboratory workflows, the required number of passages to reach the same detection threshold was calculated, assuming three population doublings per passage. This analysis, shown in FIG 5B, reflects practical constraints associated with experimental scheduling and highlights that even minor increases in cell growth are detectable within a feasible number of passages, provided cultivation is continued sufficiently.

### References

Dumont, Sampling errors and the precision associated with counting very low numbers of white cells in blood components, Transfusion (1991), 31 (5), 428-432.

Eekels et al., A competitive cell growth assay for the detection of subtle effects of gene transduction on cell proliferation, Gene Therapy (2012) 19, 1058-1064

Präbst, et al., Basic colorimetric proliferation assays: MTT, WST, and resazurin, Cell Viability Assays, Springer, 2017, pp. 1-17.

Schenone, et al., Target identification and mechanism of action in chemical biology and drug discovery, Nat. Chem. Biol. (2013), 9 (4) 232-240.

Song et al., ComProliM: A cell growth assay robust to initial cell number in co-culture system, Heliyon (2023), 9, e19433.

## Claims

1. A method of determining cell growth difference of a target cell population by performing a reciprocal read-out cell co-culture relative to at least one control cell population, comprising the following steps:
(i) providing the target cell population and the at least one control cell population as a co-culture;
(ii) expressing, preferably constitutively expressing, in the at least one control cell population at least one marker, preferably a fluorescent marker;
(iii) co-transfecting the target cell population and the at least one control cell population with at least one molecular stimulant, independently selected from a nucleic acid molecule, including a plasmid DNA, a viral vector, an RNA, an antisense oligonucleotide, a protein, a peptide, a small molecule drug, a growth factor, a cytokine, or a combination thereof, wherein the at least one molecular stimulant induces at least one genomic modification in the target cell population, preferably wherein the at least one molecular stimulant is at least one sgRNA complementary to at least one target sequence in the genome of the at least one target cell population, wherein the at least one target cell population expresses at least one Cas endonuclease and/or a variant thereof;
(iv) incubating the cell co-culture, including at least one cell passage, more preferably at least two, more preferably at least three, more preferably at least four, more preferably at least five, including at least 10, at least 20, or at least 30 cell passages;
(v) determining cell growth difference of the target cell population based on the measured marker-positive cells at a given time point post-transfection and the initial percentage of marker-positive cells at the time point of co-transfection, preferably by microscopy, live cell imaging and/or flow cytometry, preferably wherein the marker is a fluorescent marker;
(vi) optionally: obtaining a clone from the target cell population showing an increased cell growth in comparison to a control cell.

2. The method according to claim 1, wherein the target cell population constitutively or inducibly encodes at least one recombinant target molecule, preferably at least one recombinant target protein.

3. The method according to claim 1 or 2, wherein the target cell population and the at least one control cell population are co-cultivated at an equivalent initial seeding density.

4. The method according to any of the previous claims, wherein both the target cell population and the at least one control cell population are eukaryotic cell populations, preferably Chinese Hamster Ovary (CHO) cell populations.

5. The method according to any of the previous claims, wherein the at least one control cell population expresses a fluorescent marker, preferably a green-fluorescent protein (GFP), or a variant thereof, and the target cell population expresses a Cas endonuclease, or a variant thereof.

6. The method according to any of the previous claims, wherein the measurement accuracy of cell growth difference of the target cell population positively correlates with the cultivation time and/or number of cell passages relative to the at least one control cell population, according to the following relationship
(a) a minimum measurable cell growth difference of at least 30% after a cultivation time of at least three days, preferably at least four days, and/or after at least one cell passage, preferably at least two cell passages; or
(b) a minimum measurable cell growth difference of at least 20% after a cultivation time of at least five days, preferably at least six days and/or after at least two cell passages, preferably at least three cell passages; or
(c) a minimum measurable cell growth difference of at least 10% after a cultivation time of at least eight days, preferably at least 11 days and/or after at least seven cell passages, preferably at least five cell passages; or
(d) a minimum measurable cell growth difference of at least 5% after a cultivation time of at least 15 days, preferably at least 20 days and/or after at least seven cell passages, preferably at least nine cell passages; or
(e) a minimum measurable cell growth difference of at least 4% after a cultivation time of at least 19 days, preferably at least 25 days and/or after at least eight cell passages, preferably at least 11 cell passages; or
(f) a minimum measurable cell growth difference of at least 3% after a cultivation time of at least 25 days, preferably at least 33 days and/or after at least 11 cell passages, preferably at least 15 cell passages; or
(g) a minimum measurable cell growth difference of at least 2% after a cultivation time of at least 38 days, preferably at least 49 days and/or after at least 17 cell passages, preferably at least 22 cell passages; or
(h) a minimum measurable cell growth difference of at least 1% after a cultivation time of at least 63 days, preferably at least 97 days and/or after at least 32 cell passages, preferably at least 43 cell passages.

7. The method according to any of the previous claims, wherein the cell co-culture is maintained in a high-throughput manner, preferably based on an automated well-plate system.

8. The method according to any of the previous claims, wherein the target cell population and the at least one control cell population are co-transfected by high-throughput electroporation.

9. A method of screening for at least one clone within target cell population, wherein the at least one clone has an increased cell growth difference, wherein the method comprises performing a reciprocal read-out cell co-culture as defined in any of the previous claims to determine at least one clone having an increased cell growth difference; wherein step (iii) of claim 1 additionally comprises an initial step of selecting at least one molecular stimulant suitable to induce increased cell growth of the target cell population, in particular inducing increased cell growth of the target cell population.

10. A clone from the target cell population obtained or obtainable by the method according to claims 1 to 8, or obtained or obtainable by the method according to claim 9, preferably wherein the target cell population exhibits an cell growth difference rate of at least 50%, 40%, 30%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, compared to the at least one control cell population.

11. The clone according to claim 10, wherein at least one genomic modification in the target cell population allows for an optimized production of the at least one recombinant target molecule, preferably a recombinant target protein, and wherein said optimized production of the at least one recombinant target protein is **characterized by** an increase
(a) in the yield of the at least one recombinant protein in comparison to a reference, and/or
(b) in the yield of the at least one RNA encoding said at least one recombinant protein in comparison to a reference.

12. A kit for determining cell growth of a target cell population by performing a reciprocal read-out cell co-culture with at least one control cell population comprising:
(i) optionally: a chemically defined cell culture medium for CHO cell growth and transfection by electroporation;
(ii) at least one plasmid encoding at least one fluorescent protein, preferably GFP, under a constitutive promoter for stable expression in control cells;
(iii) a pre-validated sgRNA library targeting genes known to influence CHO cell growth;
(iv) at least one flow cytometry calibration standard; and
(v) optionally: detailed instructions for co-culturing in 96-well plates, electroporating using specified parameters, analyzing cell growth via flow cytometry, and calculating relative growth rates using provided software.

13. A use of the method according to claims 1 to 8 or according to the method of claim 9 for determining cell growth of a target cell population by performing a reciprocal read-out cell co-culture with at least one control cell population.

14. A use of the kit according to claim 12 for determining cell growth of a target cell population by performing a reciprocal read-out cell co-culture with at least one control cell population.

15. A use of the clone according to claim 10 or 11 for establishing a cell line for the production of a recombinant target molecule, preferably a recombinant target protein, most preferably a therapeutic molecule.
